# FASCICULE DE BREVET EUROPEEN

(11) **EP 2 903 591 B1**
(45) Date de publication et mention de la délivrance du brevet: **06.07.2016**
(21) Numéro de dépôt: 13766119.5
(22) Date de dépôt: 24.09.2013
(51) Int. Cl.: A61K 9/00, A61K 31/728, A61K 33/42, A61P 41/00, A61K 9/06, A61K 45/06, A61K 31/167, A61K 31/738

(54) **FORMULATION AQUEUSE STÉRILE INJECTABLE À BASE D'ACIDE HYALURONIQUE RÉTICULÉ ET D'HYDROXYAPATITE POUR USAGE THÉRAPEUTIQUE**
INJIZIERBARE STERILE WÄSSRIGE FORMULIERUNG AUF BASIS VON VERNETZTER HYALURONSÄURE UND HYDROXYAPATIT ZUR THERAPEUTISCHEN VERWENDUNG
INJECTABLE STERILE AQUEOUS FORMULATION BASED ON CROSSLINKED HYALURONIC ACID AND ON HYDROXYAPATITE, FOR THERAPEUTIC USE

(30) Priorité: 08.10.2012 FR 1259582
(43) Date de publication de la demande: 12.08.2015
(73) Titulaire: Aptissen S.A., 1228 Plan-les-Ouates (CH)
(72) Inventeur: GAVARD MOLLIARD, Samuel, F-74250 Bogève (FR)
(74) Mandataire: KATZAROV S.A.
(86) Numéro de dépôt international: PCT/EP2013/069877
(87) Numéro de publication internationale: WO 2014/056723

(56) Documents cités:
- WO-A1-2005/051446
- WO-A1-2007/009477
- WO-A1-2012/080915
- WO-A1-2013/053457
- WO-A2-99/38543
- WO-A2-2010/136694
- FR-A1- 2 938 187
- US-A- 5 922 025
- US-A1- 2006 040 894
- MALAFAYA ET AL: "Natural-origin polymers as carriers and scaffolds for biomolecules and cell delivery in tissue engineering applications", ADVANCED DRUG DELIVERY REVIEWS, ELSEVIER BV, AMSTERDAM, NL, vol. 59, no. 4-5, 9 juin 2007 (2007-06-09) , pages 207-233, XP022110905, ISSN: 0169-409X, DOI: 10.1016/J.ADDR.2007.03.012

## Description

### DOMAINE DE L'INVENTION

La présente invention a pour objet une formulation aqueuse stérile injectable résorbable, prête à l'emploi, utilisée dans des buts thérapeutiques sous forme de gel viscoélastique cohésif particulaire comprenant i) de l'acide hyaluronique réticulé, ou l'un de ses sels, à une concentration comprise entre 1% à 4% (masse/volume) ; la réticulation effectuée permettant d'obtenir un gel à base d'acide hyaluronique réticulé de structure dite cohésive, et ii) de l'hydroxyapatite, à une concentration comprise entre 10% à 70% (masse/volume), ladite hydroxyapatite étant sous forme de particules de taille moyenne inférieure ou égale à 80 µm, ou inférieure ou égale à 500 nm; ladite formulation aqueuse stérile injectable possédant des propriétés viscoélastiques telles que Tanδ à la fréquence de 1 Hz est inférieur ou égal à 0.60.

### CONTEXTE DE L'INVENTION

La présente invention se rapporte au domaine de la chirurgie chez l'Homme ou chez l'animal et en particulier de la chirurgie orthopédique, de la chirurgie dentaire ou maxillo-faciale, de la chirurgie ORL (Oto-Rhino-laryngologie), de la chirurgie urologique ou gynécologique, et de la chirurgie gastroentérologique.

De nombreux biomatériaux ont été développés en chirurgie pour combler et/ou restaurer des volumes et/ou remplacer des tissus biologiques sur une période plus ou moins longue.

Dans ces biomatériaux, il existe des solutions non résorbables comme par exemple les prothèses de hanche en matériau métallique utilisées en chirurgie orthopédique ou des solutions résorbables comme par exemples les stents biorésorbables à base de polymère utilisés en chirurgie cardiaque.

Les biomatériaux ciblant un comblement peuvent être utilisés pour une multitude d'applications thérapeutiques dans les tissus mous ou dans les tissus durs. On peut citer par exemple :
- en chirurgie maxillo-faciale : la reconstruction de la cavité orbitaire après énucléation à l'aide d'une bille en matériau céramique ;
- en chirurgie dentaire : le traitement de la parodontite par l'application de matériaux céramiques pour combler des poches parodontales ;
- en chirurgie urologique : le traitement de l'incontinence urinaire ou du reflux vésico-urétéral par injection endo-urétrale de produits de comblement comme par exemple des produits à base de collagène ;
- en chirurgie gastroentérologique: le traitement de l'incontinence fécale par injection de produits de comblement comme par exemple des produits à base de silicone ;
- en chirurgie ORL : le traitement des cordes vocales pour améliorer la phonation par injection de produits de comblement comme par exemple des produits à base de carbomère ;
- en chirurgie orthopédique : traitement des défauts osseux par utilisation d'un substitut osseux à base de céramique poreuse ;

De manière générale, on peut dire que les produits implantés ciblant un comblement permettent de traiter les tissus souhaités :
- en créant principalement un remplissage/augmentation de ces tissus sur le long terme pour les tissus mous.
- en favorisant principalement la construction/régénération de ces tissus pour les tissus durs.

Parmi les biomatériaux dits résorbables ciblant un comblement, on peut citer par exemple les produits contenant des particules d'hydroxyapatite.

L'hydroxyapatite possède une composition chimique très proche de celle de la phase minérale de l'os, ses propriétés biologiques et sa biocompatibilité en font un excellent produit de substitution osseuse. Une colonisation osseuse du substitut dépend étroitement des caractéristiques poreuses du matériau et en particulier de la taille et de la répartition des pores, ainsi que de l'interconnexion entre les macropores (nombre et dimension). Les interconnexions constituent des tunnels qui permettent le passage des cellules et de la circulation sanguine entre les pores et favorisent ainsi la formation osseuse au sein du substitut.

L'hydroxyapatite, de part son haut niveau de biocompatibilité et sa lente résorption dans l'organisme, est administrée dans des tissus bien différents et pour des applications variées sous des formes différentes comme par exemples :
- des ciments à base d'hydroxyapatite. Ces ciments sont préparés par le chirurgien en bloc opératoire (matériau malléable au cours de la préparation pendant quelques minutes) puis administrés sur la zone à traiter (matériau qui durcit in situ). Ils sont utilisés en tant que substitut osseux du fait de leur bonne ostéointégration et de leur biorésorbabilité permettant de laisser place à un os néoformé au cours du temps (WO2005/051446).
- des solutions ou gels à base d'hydroxyapatite pouvant contenir un polymère comme la carboxyméthylcellulose. Ces produits peuvent être utilisés dans différents domaines comme pour combler des déficiences parodontales en chirurgie dentaire ou traiter l'incontinence urinaire ou encore faire du comblement en chirurgie orthopédique après curetage de kystes ou de tumeurs bénignes (US 5 922 025).
- des produits à base d'hydroxyapatite se présentant sous forme de bloc, poudre, granulés utilisés en orthopédie et possédant une ostéointégration rapide, utilisé par exemple en comblement pour la reprise de hanche ou en maxillo facial, en tant que complément aux implants dentaires.

Parmi les produits à base d'hydroxyapatite, nombreux sont ceux qui ne sont pas prêts à l'emploi et qui nécessitent une préparation préalable par le chirurgien (cas des ciments à base d'hydroxyapatite qui requiert un mélange préalable d'une poudre et d'une solution, dans un temps déterminé, avant dépôt sur ou dans la zone à traiter et durcissement du ciment in situ). D'autres produits nécessitent d'être mis en forme par le chirurgien (matériau solide à « découper » à la bonne forme) avant implantation sur ou dans la zone à traiter. Des problèmes liés à la migration des particules d'hydroxyapatite sont également reportés. Ces problèmes peuvent générés des complications et/ou des pertes de performance du produit considéré. Par exemple, la migration des particules d'hydroxyapatite induit une perte de l'effet de comblement des tissus biologiques et peut potentiellement induire des effets secondaires. En effet, les particules d'hydroxyapatite peuvent notamment se concentrer dans certaines zones plus ou moins éloignées de la zone à traiter du fait des contraintes mécaniques soumises au biomatériau. La présence de particules ou la trop forte concentration en particules dans une zone non désirée peut induire des complications plus ou moins sévères.

L'acide hyaluronique (AH) est un autre biomatériau résorbable contenu dans de nombreux produits notamment pour faire du comblement. Il est utilisé sous sa forme native (non modifié chimiquement) ou encore sous forme réticulé dans de nombreux domaines thérapeutiques ou esthétiques.

L'acide hyaluronique réticulé est bien connu en dermo-esthétique, domaine pour lequel il est injecté dans ou sous le derme pour combler les rides ou restaurer le volume de différentes zones du corps pour une période de plusieurs mois. Il présente l'avantage de présenter très peu d'effets secondaires en post injection et des complications extrêmement rares sur le long terme. D'autre part, en cas de mauvaise injection, le praticien à la possibilité de corriger son traitement en injectant une solution de hyaluronidases (enzymes spécifiques de l'AH), solution qui va dégrader le produit à base de AH réticulé préalablement injecté. Les injections de AH réticulé, du fait de leur disparition progressive (résorption du polymère dans les tissus au cours du temps), doivent être répétées à intervalles réguliers, généralement de 6 à 12 mois, afin de maintenir l'efficacité du traitement. L'acide hyaluronique non réticulé, lui, à un temps de résidence court au niveau de la peau (demi-vie inférieure à une semaine), il est dégradé in vivo par différents facteurs comme la dégradation radicalaire, enzymatique, thermique et mécanique. C'est bien la réticulation qui permet de significativement accroître sa demi-vie en ralentissant les cinétiques de dégradation de l'acide hyaluronique selon les facteurs décrits ci-dessus, permettant ainsi une efficacité du traitement esthétique pouvant atteindre environ 12 mois.

L'acide hyaluronique, réticulé ou non réticulé, est utilisé dans des domaines thérapeutiques comme par exemple :
- en orthopédie, où il est administré en tant que viscosupplément dans les articulations atteintes par l'arthrose, pour réduire la douleur et augmenter la mobilité du patient traité,
- en ophtalmologie, en tant que solution viscoélastique au cours de la chirurgie de la cataracte pour créer un espace intraoculaire et protéger les tissus de l'oeil ou en tant qu'implant de drainage dans le cadre de la chirurgie du glaucome,
- en urologie, en tant que produit de comblement pour traiter l'incontinence urinaire ou fécale, ou
- en maxillo-faciale, pour reconstruire la cavité orbitaire après énucléation.

Une recherche scientifique intensive est effectuée à l'échelle mondiale afin de mettre au point des traitements à base d'acide hyaluronique possédant une performance renforcée dans le temps. L'objectif étant notamment d'avoir des produits capable de se dégrader moins rapidement dans les tissus biologiques afin de conserver un effet de comblement optimal sur une période la plus longue possible, tout en conservant un très haut niveau de sécurité des produits injectés. D'autre part, l'acide hyaluronique possède de très nombreux avantages, ce qui en fait un biomatériau de choix pour différentes applications médicales. Toutefois, il ne possède malheureusement pas de propriétés lui conférant une activité forte dans le domaine de l'ostéosynthèse pour la reconstruction osseuse, contrairement à un biomatériau comme l'hydroxyapatite.

Dans ce contexte, il est important de mettre à la disposition des praticiens des formulations biocompatibles prêtes à l'emploi et facilement administrables possédants des propriétés mécaniques remarquables et un effet long terme adaptés aux injections à buts thérapeutique sans entraîner des complications liées à une migration du produit implanté.

FR 2 938 187 divulgue déjà une composition viscoélastique injectable à base d'acide hyaluronique et comprenant un ou plusieurs polyol(s) et de la lidocaïne. Cette composition présente des propriétés rhéologiques améliorées et une longue rémanence in vivo.

### RESUME DE L'INVENTION

L'invention concerne une formulation aqueuse stérile injectable et biorésorbable, prête à l'emploi, utilisée dans des buts thérapeutiques, sous forme de gel viscoélastique cohésif particulaire comprenant i) de l'acide hyaluronique réticulé, ou l'un de ses sels, à une concentration comprise entre 1% à 4% (masse/volume) ; la réticulation effectuée permettant d'obtenir un gel à base d'acide hyaluronique réticulé de structure dite cohésive, et ii) de l'hydroxyapatite, à une concentration comprise entre 10% à 70% (masse/volume), ladite hydroxyapatite étant sous forme de particules de taille moyenne inférieure ou égale à 80 µm, ou inférieure ou égale à 500 nm; ladite formulation aqueuse stérile injectable possédant des propriétés viscoélastiques telles que Tanδ à la fréquence de 1 Hz est inférieur ou égal à 0.60.

Selon un autre objet, la présente invention concerne un procédé de préparation d'une formulation aqueuse stérile injectable comprenant les étapes consistant à : a) préparer un premier mélange comprenant au moins 1% à 4% en poids d'acide hyaluronique réticulé ou d'un de ses sels, par formation de liaisons covalentes entre les chaînes dudit biopolymère à l'aide de molécules bi- ou polyfonctionnelles, la réticulation effectuée permettant d'obtenir un gel à base d'acide hyaluronique réticulé de structure dite cohésive, b) purifier ledit premier mélange, c) ajouter ensuite l'hydroxyapatite à une concentration comprise entre 10% à 70% (masse/volume) en la dispersant de manière homogène dans le gel à base d'acide hyaluronique réticulé, d) mettre le gel ainsi obtenu sous forme prête à l'emploi, e) stériliser le produit à la chaleur humide.

Selon un autre objet également, la présente invention concerne un kit se présentant de préférence sous la forme de seringue, ampoule ou flacon contenant la formulation telle que décrite précédemment.

### BREVE DESCRIPTION DE LA FIGURE

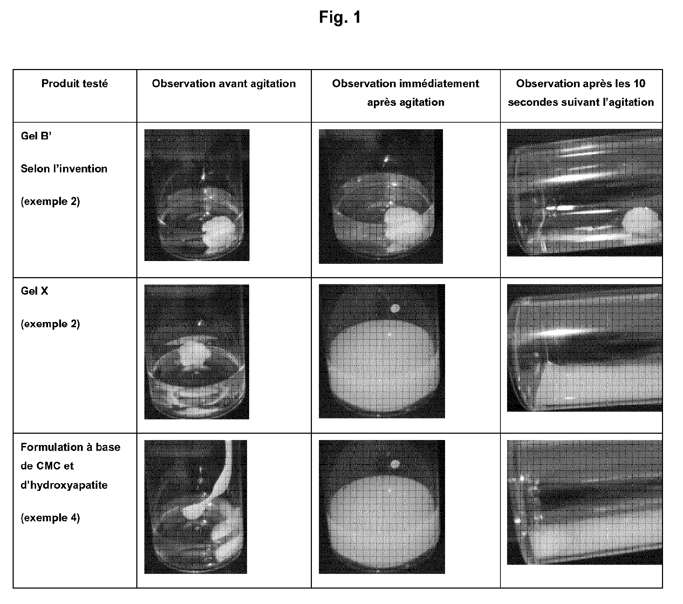

**La** **figure 1** représente des photos de la comparaison des gels B', X et de la formulation à base de CMC et d'hydroxyapatite, selon le test décrit dans l'exemple 2.

### DESCRIPTION DETAILLEE DE L'INVENTION

L'invention décrite ci-après a pour objectif de proposer une nouvelle formulation aqueuse stérile injectable biorésorbable, prête à l'emploi, utilisée dans des buts thérapeutiques et présentant des propriétés spécifiques de viscoélasticité, de comblements, de performance à long terme et dans certains cas d'une capacité à favoriser activement la restauration des tissus biologiques environnants. Cette formulation est caractérisée en ce qu'elle est sous forme de gel viscoélastique cohésif particulaire comprenant
i) de l'acide hyaluronique réticulé, ou l'un de ses sels, à une concentration comprise entre 1% à 4% (masse/volume) ; la réticulation effectuée permettant d'obtenir un gel à base d'acide hyaluronique réticulé de structure dite cohésive, et
ii) de l'hydroxyapatite, à une concentration comprise entre 10% à 70% (masse/volume), ladite hydroxyapatite étant sous forme de particules de taille moyenne inférieure ou égale à 80 µm, ou inférieure ou égale à 500 nm;
ladite formulation aqueuse stérile injectable possédant des propriétés viscoélastiques telles que Tanδ à la fréquence de 1 Hz est inférieure ou égal à 0.60.

La nouvelle formulation de la présente invention est biorésorbable. Elle comprend des biomatériaux qui sont biocompatibles et assimilables par les tissus biologiques à plus ou moins long terme. Dans de nombreuses applications, et notamment dans le domaine de la reconstruction osseuse en orthopédie, elle peut jouer le rôle de promoteur de la repousse des tissus biologiques environnants. Dans le cas des applications en reconstruction osseuse, la formulation selon l'invention se résorbe et laisse place à un os néoformé au cours du temps.

De manière tout à fait surprenante, il a été constaté que cette formulation possède une capacité remarquable pour le comblement et/ou la restauration de volumes et/ou le remplacement de tissus biologiques sur le long terme, ceci grâce à une synergie entre l'acide hyaluronique réticulé et les particules d'hydroxyapatite, selon les conditions de l'invention.

D'un point de vue mécanique, les particules d'hydroxyapatite (de comportement solide : forte élasticité et viscosité négligeable) renforcent considérablement l'élasticité du gel et donc sa capacité à créer du volume en induisant une force/pression importante sur les tissus pour corriger la zone déficiente à traiter.

L'acide hyaluronique réticulé apporte des propriétés de viscoélasticité, c'est-à-dire d'élasticité mais également de viscosité permettant d'avoir une consistance de gel se rapprochant de celle des tissus mous et donc ainsi de contrebalancer la très forte élasticité et l'absence de viscosité apportée par les particules d'hydroxyapatite. Ceci permettant d'avoir un produit s'intégrant dans les tissus de manière beaucoup plus homogène, moins traumatique pour les tissus (forte limitation de l'inflammation en post injection) et moins douloureux à l'injection.

D'autre part, l'acide hyaluronique réticulé, dans les conditions de l'invention, va permettre de réduire considérablement la migration des particules d'hydroxyapatite, particules qui sont retenues au sein du gel, de part la forte cohésivité apportée par l'acide hyaluronique réticulé de nature cohésive (acide hyaluronique réticulé possédant une faible cinétique de résorption). Cette forte limitation de la migration permet d'avoir :
- un gel avec une capacité volumatrice améliorée sur le long terme,
- une réduction des effets secondaires en permettant aux particules d'hydroxyapatite de rester sur le site d'injection.

L'acide hyaluronique est un polysaccharide composé de la répétition d'unités disaccharide de glucuronate et de N-acetyl glucosamine. Il est réparti largement parmi les tissus conjonctifs, épithéliaux et nerveux chez les humains comme chez les animaux. Il constitue l'un des principaux composants de la matrice extracellulaire. Il contribue de façon significative à la prolifération et à la migration des cellules. Il est notamment trouvé en concentration importante dans l'humeur aqueuse, le liquide synovial, la peau et le cordon ombilical.

Parmi les sels d'acide hyaluronique préférés selon l'invention, on citera les sels d'acide hyaluronique, avec un cation, par exemple un sel mono- ou divalent tel que les sels de sodium, potassium, magnésium, calcium, manganèse. Les sels de sodium sont tout particulièrement préférés.

Selon l'invention, l'acide hyaluronique ou d'un de ses sels, est sous forme réticulée. Cette réticulation est obtenue par formation de liaisons covalentes entre les chaînes dudit biopolymère à l'aide de molécules bi- ou polyfonctionnelles, la réticulation effectuée permettant d'obtenir un gel à base d'acide hyaluronique réticulé de structure dite cohésive, encore appelée monophasique.

Dans un exemple non limitatif de la présente invention, l'acide hyaluronique réticulé est fabriqué à partir de fibres de hyaluronate de sodium mises en contact avec du butanediol diglycidyl ether (BDDE) pour former un réseau gélifié.

La nature cohésive du gel à base d'acide hyaluronique réticulé est une spécificité majeure et nécessaire de l'invention. Le gel ne doit pas se désagréger rapidement lorsqu'on l'introduit dans de l'eau, comme le fait un gel de nature non cohésive, encore appelé gel biphasique (type de gel à base d'acide hyaluronique réticulé n'étant pas en mesure de maintenir les particules d'hydroxyapatite et donc d'éviter la migration). L'exemple 2 met en avant cette différence entre un gel cohésif et un gel non cohésif.

La présente invention comprend une concentration en acide hyaluronique réticulé, ou de l'un de ses sels, comprise entre 1% à 4% (masse/volume), de préférence entre 1% et 3% (masse/volume). Selon une variante particulièrement préférée, la concentration en acide hyaluronique réticulé, ou de l'un de ses sels, est comprise entre 1.5% à 2.5% (masse/volume). Alternativement, la concentration en acide hyaluronique réticulé, ou de l'un de ses sels, peut être comprise entre 1.5% à 3% (masse/volume), ou 1% à 2.5% (masse/volume).

Avantageusement, la formulation aqueuse selon l'invention comprend de l'acide hyaluronique, ou l'un de ses sels, dont la masse moléculaire est préférablement comprise entre 2.5x10⁵ Da et 4x10⁶ Da. Selon une variante particulièrement préférée, cette masse moléculaire est comprise entre 1x10⁶ Da et 3x10⁶. Alternativement, la masse moléculaire est comprise entre 1x10⁶ Da et 2.5x10⁶, ou 2.5x10⁵ Da et 3x10⁶ Da.

L'hydroxyapatite est une espèce minérale de la famille des phosphates, de formule Ca5(PO4)3(OH), usuellement écrite Ca10(PO4)6(OH)2 pour souligner le fait que la maille de la structure cristalline comprend 2 molécules. L'hydroxyapatite appartient à la famille cristallographique des apatites, composés isomorphes possédant une même structure hexagonale. Ce composé est utilisé comme biomatériau depuis de nombreuses années dans différentes spécialités médicales.

La présente invention comprend une concentration en particules d'hydroxyapatite comprise entre 10 à 70% (masse/volume), de préférence entre 20 à 60% (masse/volume), de préférence entre 30 à 50% (masse/volume) et la taille moyenne des particules d'hydroxyapatite est inférieure ou égale à 80 µm, ou inférieure ou égale à 500 nm.

Il a été observé que les propriétés de viscosité et d'élasticité de la formulation selon invention sont optimales lorsque le paramètre Tan delta ou Tanδ, correspondant au rapport [module visqueux G" /module élastique G'] à la fréquence de 1 Hz, est inférieur ou égal à 0.60, de préférence inférieur ou égal à 0.58. En effet, il a été montré que le caractère élastique de la formulation selon l'invention, par rapport à sa viscosité, doit être suffisamment important pour pouvoir éviter la sédimentation des particules d'hydroxyapatite. Ainsi, il a été observé qu'en dessus de 0.60, les particules d' hydroxyapatite ont tendance à sédimenter au cours du temps. Cette sédimentation implique l'obtention d'une formulation à base de particules d'hydroxyapatite non homogène, ce qui n'est pas satisfaisant d'une part pour l'acte d'injection de la formulation à travers l'aiguille (bouchage d'aiguille) et d'autre part pour la sécurité et la performance de la formulation au niveau de la zone d'injection (par exemple, création de zones dites dures dans les tissus induites par la concentration de particules d'hydroxyapatite dans certaines zones plus ou moins éloignées de la zone à traiter du fait des contraintes mécaniques soumises au biomatériau. La présence de particules ou la trop forte concentration en particules dans une zone non désirée pouvant induire des complications plus ou moins sévères).

En général, la mesure de l'élasticité (G') et du rapport de la viscosité sur l'élasticité (Tan delta=G"/G') est effectuée en réalisant un balayage en fréquence de 0.01 à 100 Hz à l'aide d'un rhéomètre avec une géométrie plate de 40 mm, un entrefer de 1000 micromètres et une température d'analyse de 25°C.

Comme montré dans l'exemple 2, la cohésivité de la formulation selon l'invention est un élément majeur mais il est également nécessaire que le caractère viscoélastique de celui-ci soit approprié afin :
- d'éviter la sédimentation au cours du temps des particules d'hydroxyapatite au sein de leur contenant, et
- d'éviter d'avoir un produit qui va se séparer en 2 phases (particules d'hydroxyapatite et gel d'acide hyaluronique réticulé) lors de l'injection et/ou au niveau de la zone d'injection, créant ainsi des hétérogénéités au niveau de la zone traitée.

L'invention a également pour objectif de présenter une meilleure longévité au cours du temps en comparaison avec les formulations de l'art antérieur. Cette meilleure longévité du comblement de la zone à traiter est obtenue grâce à la capacité de l'acide hyaluronique réticulé à maintenir sur le long terme les particules d'hydroxyapatite au niveau de la zone d'injection et aux particules d'hydroxyapatite à conférer des propriétés mécaniques/rhéologiques remarquables sur le long terme. Le gain sur la longévité en clinique étant probablement de plusieurs mois.

Il est également important de préciser que la présence des particules d'hydroxyapatite, radio-opaques, confère un avantage au gel car pouvant être localisé aisément par le praticien sous radiographie pendant et/ou après l'injection.

La possibilité donnée au praticien d'injecter une solution de hyaluronidases pour corriger son injection et dégrader l'acide hyaluronique réticulé composant le produit confère également un avantage à l'invention. Cette injection ne permet néanmoins pas d'accélérer la résorption des particules d'hydroxyapatite : il n'y a donc pas dégradation complète du produit au sein des tissus.

La présente invention consiste donc en une formulation, telle que décrite ci-dessus, utilisée pour le comblement et/ou la restauration de volumes et/ou le remplacement et/ou la régénération de tissus biologiques, comme par exemple :
- en chirurgie maxillo-faciale pour la reconstruction de la cavité orbitaire ;
- en chirurgie dentaire pour le traitement de la parodontite ;
- en chirurgie urologique pour le traitement de l'incontinence urinaire ou du reflux vésico-urétéral par injection endo-urétrale ;
- en chirurgie gastroentérologique pour le traitement de l'incontinence fécale ;
- en chirurgie ORL pour le traitement des cordes vocales pour améliorer la phonation ;
- en chirurgie orthopédique pour le traitement des défauts osseux et
- en chirurgie gynécologique.

La formulation selon l'invention est généralement utilisée telle quelle mais il n'est pas exclu qu'il lui soit ajouté au moins un autre additif (autre que ceux citées plus haut) et/ou au moins un principe actif.

Ainsi, la formulation peut comprendre en outre un ou plusieurs matériaux céramiques. Ces matériaux sont généralement choisis parmi le groupe comprenant le phosphate tricalcique, le carbonate de calcium et le sulfate de calcium, ou une combination de plusieurs de ses matériaux céramiques.

La formulation selon l'invention peut comprendre en outre un ou plusieurs facteurs de croissance, comme ceux de la famille des « Protéines morphogénétique osseuses » (BMPs) et/ou des « facteurs de croissance transformant b » (TGF-βs). Dans la grande famille des facteurs de croissance TGF β, les facteurs de croissance morphogènes (BMPs) ont une action plus spécifique sur l'ostéogénèse. Les BMPs sont capable d'induire la formation d'os, ce sont des biomatériaux osteoinducteurs. Ils sont présents en quantité infinitésimale dans le squelette (1 µg/kg d'os). Le développement de la biologie moléculaire et en particulier des techniques de clonage ont permis de produire ces facteurs en quantité illimitée et très pure par génie génétique sous la forme recombinante rhBMP-2.

La formulation selon l'invention peut également comprendre en outre un ou plusieurs anesthésiques, choisi parmi le groupe comprenant la lidocaïne seule ou en combinaison avec de l'adrénaline, la procaïne, l'étidocaïne seule ou en combinaison avec de l'adrénaline, l'articaïne seule ou en combinaison avec de l'adrénaline, la mépivacaïne, la pramocaïne, la quinisocaïne, ou un ou plusieurs des sels de ces anesthésiques. Selon une variante particulièrement préférée, l'anesthésique choisi est le chlorhydrate de lidocaïne. La présence d'un anesthésique dans la formulation selon l'invention présente un intérêt majeur pour l'amélioration du confort du patient au cours et après injection.

Selon un autre mode particulier de l'invention, la formulation selon l'invention peut également comprendre en outre un ou plusieurs antioxydants, comme les antioxydants de la famille des polyols. L'antioxydant pourra être choisi parmi le groupe des polyols comprenant notamment le sorbitol, le glycérol, le mannitol ou le propylène glycol.

Selon un autre objet, la présente invention concerne un procédé de préparation d'une formulation aqueuse stérile injectable comprenant les étapes consistant à : a) préparer un premier mélange comprenant au moins 1% à 4% en poids d'acide hyaluronique réticulé ou d'un de ses sels, par formation de liaisons covalentes entre les chaînes dudit biopolymère à l'aide de molécules bi- ou polyfonctionnelles, la réticulation effectuée permettant d'obtenir un gel à base d'acide hyaluronique réticulé de structure dite cohésive, b) purifier ledit premier mélange, c) ajouter ensuite l'hydroxyapatite à une concentration comprise entre 10% à 70% (masse/volume) en la dispersant de manière homogène dans le gel à base d'acide hyaluronique réticulé, d) mettre le gel ainsi obtenu sous forme prête à l'emploi, e) stériliser le produit à la chaleur humide.

La stérilisation de la formulation selon l'étape e) est réalisée à la chaleur humide. L'homme de l'art saura sélectionner un cycle de stérilisation à la chaleur (température et durée du cycle de stérilisation) approprié à la stérilisation de son produit. Par exemple, les cycles de stérilisation à la chaleur humide suivants peuvent être utilisés : 131 °C, 1 min / 130°C, 3 min / 125°C, 7 min / 121 °C, 20 min.

Selon un autre objet, la présente invention concerne un kit se présentant de préférence sous la forme de seringue contenant la formulation telle que décrite précédemment.

De façon avantageuse, la formulation est prête à l'emploi, elle peut être injectée directement par le praticien. Le type d'injection dépend du site et/ou des tissus biologiques considérés. L'invention peut être utilisée pour de nombreuses applications thérapeutiques notamment :
- en chirurgie maxillo-faciale pour la reconstruction de la cavité orbitaire ;
- en chirurgie dentaire pour le traitement de la parodontite ;
- en chirurgie urologique pour le traitement de l'incontinence urinaire ou du reflux vésico-urétéral par injection endo-urétrale ;
- en chirurgie gastroentérologique pour le traitement de l'incontinence fécale ;
- en chirurgie ORL pour le traitement des cordes vocales pour améliorer la phonation ;
- en chirurgie orthopédique pour le traitement des défauts osseux et
- en chirurgie gynécologique.

La présente invention concerne également un kit sous forme d'un contenant différent d'une seringue comme une ampoule ou un flacon contenant la formulation telle que décrit précédemment.

L'invention va maintenant être illustrée à titre non limitatif par les exemples 1 à 4 suivants :

### EXEMPLES

### Exemple 1

### Préparation d'un gel à base d'acide hyaluronique réticulé de structure dite cohésive

Etape 1 : 3.5 g de hyaluronate de sodium de poids moléculaire 2,6 MDa sont ajoutés à de la soude 1 % (30,5 g). Le mélange est laissé à l'homogénéisation pendant 1 h 30. 420 mg de butanediol diglycidyl ether (BDDE) sont ajoutés au mélange qui est homogénéisé, fermé puis placé au bain-marie à 50°C pendant 2 h. Le mélange est ensuite neutralisé par l'ajout de 7.5 g de HCl 1N.

Le gel est purifié pendant 24h par dialyse avec une solution physiologique iso-osmolaire et possédant un pH neutre (cellulose régénérée, limite de séparation : masse moléculaire = 60 kDa) afin d'obtenir une concentration en acide hyaluronique de 25 mg/ml (2.5%). Il est ensuite homogénéisé dans un mélangeur classique à pâles pendant 1h30 (= gel A1 / 124 g).

Le gel peut enfin être dégazé, rempli en seringues en verre de 2 ml et stérilisé par autoclavage vapeur à 130°C pendant 3 minutes (= gel A / gel viscoélastique de structure dite cohésive ou monophasique).

**Etape 2 :** préparation du gel selon l'invention. Dans 100g de gel A1, on ajoute 42.9 g de phosphocalcium hydroxyapatite Ca10(PO4)6(OH)2 dont les particules ont une granulométrie moyenne comprise entre 30 et 50 micromètres puis on homogénéise le gel dans un mélangeur classique à pâles pendant 1h30 (= gel B1 / 142.9g). Le gel peut enfin être dégazé, rempli en seringues en verre de 2 ml et stérilisé par autoclavage vapeur à 130°C pendant 3 minutes (= gel B).
Le gel est viscoélastique cohésif particulaire. En effet, celui-ci se présente sous la forme d'un gel viscoélastique (il présente des propriétés d'élasticité G' et de viscosité G" / voir ci-dessous), possédant une forte cohésivité (voir exemple 2) et contenant des particules d'hydroxyapatite.
La concentration en acide hyaluronique du gel est de 17.5 mg/ml (1.75%) (dosage au carbazole, méthode de la Pharmacopée Européenne). D'autre part, le pH (7.15) et l'osmolarité (315 mOsm/kg) du gel sont physiologiques.
Le gel est facilement injectable à travers une aiguille : Une force de 26.3 N est nécessaire pour pousser le gel à travers une aiguille de 21 G, en considérant une vitesse de poussée de 12.5 mm/minute.

Les gels A et B sont caractérisés d'un point de vue mécanique/rhéologique : Le rhéomètre utilisé pour effectuer ces caractérisations est un AR2000 (TA instruments) avec une géométrie plate de 40 mm, un entrefer de 1000 micromètres est une température d'analyse de 25°C.

Une mesure de l'élasticité (G') et du rapport de la viscosité sur l'élasticité (Tan delta=G"/G') est effectuée en réalisant un balayage en fréquence de 0.01 à 100 Hz. Une comparaison des paramètres est effectuée à 1 Hz.

| **Gel** | **G'(1 Hz) en Pa** | **Tan delta (1 Hz)** |
|---|---|---|
| A | 184 | 0.25 |
| B | 381 | 0.29 |

On constate que le produit B possède une élasticité significativement plus forte que le produit A. Le Tan delta des 2 produits A et B, quant à lui, est relativement proche : le gel B conserve un caractère visqueux important, malgré la présence des particules d'hydroxyapatite (qui elles possèdent une forte élasticité et une viscosité négligeable).

Une mesure de la force normale induite par le gel à tester est effectuée par compression de l'échantillon entre le plan peltier et la géométrie pour un entrefer de 1500 micromètres et une quantité de gel de 1.4 g.

| Gel | Force normale (N) |
|---|---|
| A | 0.86 |
| B | 1.47 |

On constate que le produit B possède une élasticité et une force normale induite significativement plus forte que le produit A.

Cette plus forte élasticité, en combinaison avec la forte cohésivité du gel selon l'invention, confère une meilleure capacité du produit à créer du volume dans les tissus.

### Exemple 2

### Importance de la structure dite cohésive du gel à base de HA réticulé - Comparatif

Le gel A1 (de structure dite cohésive ou monophasique) décrit dans l'exemple 1 est dialysé avec une solution physiologique iso-osmolaire et possédant un pH neutre (cellulose régénérée, limite de séparation : masse moléculaire = 60 kDa) afin d'obtenir une concentration en acide hyaluronique de 20 mg/ml (2%).

De l'hydroxyapatite de calcium est ensuite ajouté dans le gel afin d'obtenir une concentration de 200 mg/ml (20%) puis un mélange à la spatule est effectué (2 minutes pour 5g de gel).

Le gel ainsi obtenu est ensuite stérilisé à l'autoclave à 121°C pendant 20 minutes (= gel B' selon l'invention).

Le gel commercial Restylane® Perlane® (lot 11363-1) à base d'acide hyaluronique réticulé de structure dite non cohésive ou biphasique, dont la concentration en acide hyaluronique est de 20 mg/ml (2%) est dopé avec 200 mg/ml (20%) d'hydroxyapatite de calcium par mélange à la spatule (2 minutes pour 5g de gel).

Le gel ainsi obtenu est ensuite stérilisé à l'autoclave à 121 °C pendant 20 minutes (= gel X).

Le gel A1 et le gel Restylane® Perlane® sont comparés selon le test suivant :
Dans des flacons en plastique de 30 ml contenant 5 ml d'eau purifiée, on introduit 1 ml de gel A1 dans le flacon 1 et 1 ml de gel Restylane® Perlane® dans le flacon 2. Après fermeture des flacons, on mélange manuellement les 2 flacons pendant 5 secondes. Après 10 secondes, on observe que le gel Restylane® Perlane® s'est complètement désagrégé/dispersé sous forme d'une multitude de particules dans la solution aqueuse. Le gel Restylane® Perlane® possède donc bien une structure dite non cohésive ou biphasique (le gel se disperse rapidement dans la solution aqueuse).

Le gel A1, lui, est toujours sous la forme d'une « boule de gel » dans la solution aqueuse. Il possède donc bien une structure dite cohésive ou monophasique (le gel ne se disperse pas rapidement dans la solution aqueuse, il possède une forte cohésivité, contrairement au gel Restylane® Perlane®).

Le gel B' selon l'invention et le gel X sont comparés selon le test suivant (voir figure 1): Dans des flacons en plastique de 30 ml contenant 5 ml d'eau purifiée, on introduit 1 ml de gel B' dans le flacon 1 et 1 ml de gel X dans le flacon 2. Après fermeture des flacons, on mélange manuellement les 2 flacons pendant 5 secondes. Après 10 secondes, on observe que le gel X s'est complètement désagrégé/dispersé sous forme d'une multitude de particules dans la solution aqueuse. Le gel X possède une structure viscoélastique non cohésive particulaire. Il ne correspond pas aux caractéristiques du gel selon l'invention. En pratique médicale pour une utilisation en esthétique, celui-ci va diffuser/migrer au niveau de la zone d'injection.

Le gel B', lui, est toujours sous la forme d'une « boule de gel » dans la solution aqueuse. Il possède donc bien une structure cohésive particulaire qui va permettre, dans le cadre de la pratique médicale pour une utilisation en esthétique, de ne pas diffuser/migrer au niveau de la zone injectée, et ainsi d'éviter les complications liées à la migration de particules d'hydroxyapatite dans les tissus mais également d'avoir une meilleure performance long terme du produit car le gel injecté va pouvoir maintenir sa capacité à créer du volume dans les tissus sur une longue période, du fait de l'absence de migration du biomatériau de la zone traitée.

### Exemple 3

### Importance de la viscoélasticité du gel selon l'invention - Comparatif

Soit C un gel préparé selon le même protocole (étapes 1&2) que celui décrit en exemple 1 en introduisant 200 mg de BDDE au lieu de 420 mg.

Soit D un gel préparé selon le même protocole (étapes 1&2) que celui décrit en exemple 1 en introduisant 290 mg de BDDE au lieu de 420 mg.

Le gel C est caractérisé d'un point de vue mécanique/rhéologique.

Le rhéomètre utilisé pour effectuer les caractérisations rhéologiques est un AR2000 (TA instruments) avec une géométrie plate de 40 mm, un entrefer de 1000 micromètres est une température d'analyse de 25°C.

Une mesure du rapport de la viscosité sur l'élasticité (Tan delta=G"/G') est effectuée en réalisant un balayage en fréquence de 0.01 à 100 Hz.

Une comparaison des paramètres est effectuée à 1 Hz.

| **Gel** | **Tan delta = G"/G'(1 Hz)** |
|---|---|
| C | 0.84 |
| D | 0.58 |

On constate que les particules d'hydroxyapatite ont tendance à sédimenter au cours du temps (phénomène bien observé par passage d'un échantillon à la centrifugeuse) pour le gel C, ce qui n'est pas observé pour le gel D.

Cette sédimentation implique l'obtention de produits à base de particules d'hydroxapatite non homogène, ce qui n'est pas satisfaisant pour l'acte d'injection du gel à travers l'aiguille (bouchage de l'aiguille) mais également pour la sécurité et la performance du produit au niveau de la zone d'injection (risques importants de complications comme par exemple la création de zones dites dures).

Comme montré dans l'exemple 2, la cohésivité du gel selon l'invention est importante mais il est également nécessaire que le caractère viscoélastique de celui-ci soit approprié afin :
- d'éviter la sédimentation au cours du temps des particules d'hydroxyapatite au sein de leur contenant
- d'éviter d'avoir un produit qui va se séparer en 2 phases (particules d'hydroxyapatite et gel d'acide hyaluronique réticulé) lors de l'injection et/ou au niveau de la zone d'injection, créant ainsi des hétérogénéités au niveau de la zone traitée.

Le caractère élastique du gel (par rapport à sa viscosité) doit donc être suffisamment important pour pouvoir éviter la sédimentation des particules.

### Exemple 4

### Comparaison d'un gel selon l'invention avec des solutions de l'art antérieur

### a) Formulation à base de HA non réticulé et d'hydroxyapatite

Comme décrit dans la littérature, in vivo, l'acide hyaluronique non réticulé a une demi-vie inférieure à une semaine.

Par conséquent, une solution de HA non réticulé avec hydroxyapatite n'est pas intéressante car l'acide hyaluronique non réticulé va se résorber très rapidement et il ne va pas permettre d'empêcher la migration des particules d'hydroxyapatite sur le long terme.

### b) Formulation aqueuse d'hydroxyapatite

Une solution aqueuse d'hydroxyapatite (S1) est préparée (30% de phosphocalcium hydroxyapatite possédant une granulométrie comprise entre 30 et 50 micromètres dans une solution physiologique iso-osmolaire et possédant un pH neutre).

Dans un flacon en plastique de 30 ml contenant 5 ml d'eau purifiée, on introduit 1 ml de la solution S1. On observe une dispersion immédiate des particules d'hydroxyapatite dans le flacon.

Contrairement à la formulation selon l'invention, la solution S1 n'est pas en mesure de maintenir les particules d'hydroxyapatite au niveau de la zone d'injection sur le long terme.

### c) Formulation à base de CMC et d'hydroxyapatite (voir figure 1).

Une formulation aqueuse de carboxyméthylcellulose (CMC) et d'hydroxyapatite (S3) est préparée (30% de phosphocalcium hydroxyapatite possédant une granulométrie comprise entre 30 et 50 micromètres, et 2% de CMC à 250 000 Da dans une solution physiologique iso-osmolaire et possédant un pH neutre).

Dans un flacon en plastique de 30 ml contenant 5 ml d'eau purifiée, on introduit 1 ml de la formulation S3. Après fermeture du flacon, on mélange manuellement le flacon pendant 5 secondes.

Après 10 secondes, on observe que la formulation S3 s'est complètement désagrégée/dispersée sous forme d'une multitude de particules dans la solution aqueuse.

Contrairement à la formulation selon l'invention, la formulation S3 n'est pas en mesure de maintenir les particules d'hydroxyapatite au niveau de la zone d'injection sur le long terme.

## Revendications

1. Formulation aqueuse stérile injectable, stérilisée à la chaleur humide, utilisée dans des buts thérapeutiques, sous forme de gel viscoélastique cohésif particulaire comprenant
i) de l'acide hyaluronique réticulé, ou l'un de ses sels, à une concentration comprise entre 1% à 4% (masse/volume) ; la réticulation effectuée permettant d'obtenir un gel à base d'acide hyaluronique réticulé de structure dite cohésive, et
ii) de l'hydroxyapatite, à une concentration comprise entre 10% à 70% (masse/volume), ladite hydroxyapatite étant sous forme de particules dont la taille moyenne est inférieure ou égale à 80 µm, ou inférieure ou égale à 500 nm ;
ladite formulation aqueuse stérile injectable possédant des propriétés viscoélastiques telles que Tanδ à la fréquence de 1 Hz est inférieur ou égal à 0.60 tel que déterminé en réalisant un balayage en fréquence de 0.01 à 100 Hz à l'aide d'un rhéomètre avec une géométrie plate de 40 mm, un entrefer de 1000 micromètres et une température d'analyse de 25°C.

2. La formulation aqueuse stérile injectable selon la revendication 1, **caractérisée en ce que** la masse moléculaire de l'acide hyaluronique, ou de l'un de ses sels, est comprise entre 2.5x10⁵Da et 4x10⁶Da.

3. La formulation aqueuse stérile injectable selon la revendication 1, **caractérisée en ce que** la concentration de l'acide hyaluronique réticulé, ou de l'un de ses sels, est comprise entre 1% et 3% (masse/volume).

4. La formulation aqueuse stérile injectable selon revendications 1 à 3, **caractérisée en ce que** la concentration de l'hydroxyapatite est comprise entre 20% à 60% (masse/volume), de préférence entre 30% à 50%.

5. La formulation aqueuse stérile injectable selon l'une quelconque des revendications précédentes, **caractérisée en ce que** la formulation comprend en outre un ou plusieurs matériaux céramiques comme le tricalcium phosphate.

6. La formulation aqueuse stérile injectable selon la revendication 5, **caractérisée en ce que** le matériau céramique est le tricalcium phosphate.

7. La formulation aqueuse stérile injectable selon l'une quelconque des revendications précédentes, **caractérisée en ce que** la formulation comprend en outre un ou plusieurs anesthésiques.

8. La formulation aqueuse stérile injectable selon la revendication 7, **caractérisée en ce que** l'un ou plusieurs anesthésiques est choisi parmi le groupe comprenant la lidocaïne seule ou en combinaison avec de l'adrénaline, la procaïne, l'étidocaïne seule ou en combinaison avec de l'adrénaline, l'articaïne seule ou en combinaison avec de l'adrénaline, la mépivacaïne, la pramocaïne, la quinisocaïne, ou un ou plusieurs des sels de ces anesthésiques.

9. La formulation aqueuse stérile injectable selon la revendication 8, **caractérisée en ce que** l'anesthésique est le chlorhydrate de lidocaine.

10. La formulation aqueuse stérile injectable selon l'une quelconque des revendications précédentes, **caractérisée en ce que** la formulation comprend en outre un ou plusieurs antioxydants.

11. La formulation aqueuse stérile injectable selon la revendication 10, **caractérisée en ce que** l'un ou plusieurs antioxydants est choisi parmi la famille des polyols.

12. La formulation aqueuse stérile injectable selon la revendication 10, **caractérisée en ce que** les polyols comprennent le sorbitol, le glycérol, le mannitol ou le propylène glycol.

13. La formulation aqueuse stérile injectable selon l'une quelconque des revendications précédentes, **caractérisée en ce que** la formulation comprend en outre un ou plusieurs facteurs de croissance, comme ceux de la famille des « Protéines morphogénétique osseuses » (BMPs) et/ou des « facteurs de croissance transformant β » (TGF-βs).

14. La formulation aqueuse stérile injectable selon l'une quelconque des revendications précédentes, utilisée en chirurgie orthopédique, dentaire, maxillo-faciale ORL, gastroentérologique, urologique ou gynécologique.

15. La formulation aqueuse stérile injectable selon l'une quelconque des revendications précédentes, utilisée pour le comblement et/ou la restauration de volumes et/ou le remplacement de tissus biologiques.

16. Kit contenant la formulation aqueuse stérile injectable, selon l'une quelconque des revendications 1 à 15.

17. Kit selon la revendication 16 se présentant sous forme de seringue, ampoule ou flacon.

18. Procédé de fabrication d'une formulation aqueuse stérile injectable comprenant les étapes consistant à : a) préparer un premier mélange comprenant au moins 1% à 4% en poids d'acide hyaluronique réticulé ou d'un de ses sels, par formation de liaisons covalentes entre les chaînes dudit biopolymère à l'aide de molécules bi- ou polyfonctionnelles, la réticulation effectuée permettant d'obtenir un gel à base d'acide hyaluronique réticulé de structure dite monophasique ou cohésive, b) purifier ledit premier mélange, c) ajouter ensuite l'hydroxyapatite à une concentration comprise entre 10% à 70% (masse/volume) en la dispersant de manière homogène dans le gel à base d'acide hyaluronique réticulé ladite hydroxyapatite étant sous forme de particules dont la taille moyenne est inférieure ou égale à 80 µm, ou inférieure ou égale à 500 nm, d) mettre le gel ainsi obtenu sous forme prête à l'emploi, e) stériliser le produit à la chaleur humide.

## Patentansprüche

1. Injizierbare sterile wässrige Formulierung, unter feuchter Hitze sterilisiert, zu therapeutischen Zwecken verwendet, in der Form eines partikulären kohäsiven viskoelastischen Gels, mit:
i) vernetzter Hyaluronsäure, oder einem ihrer Salze, in einer Konzentration zwischen 1% und 4% (Masse/Volumen), wobei die durchgeführte Vernetzung es ermöglicht, ein Gel auf Basis von vernetzter Hyaluronsäure mit einer so-genannten kohäsiven Struktur zu erhalten; und
ii) Hydroxyapatit, in einer Konzentration zwischen 10% und 70% (Masse/ Volumen), wobei das besagte Hydroxyapatit in der Form von Partikeln vorliegt, deren Durchschnittsgrösse weniger als oder gleich 80µm oder weniger als oder gleich 500nm beträgt;
worin die besagte injizierbare sterile wässrige Formulierung mit viskoelastischen Eigenschaften wie Tanδ bei der Frequenz von 1Hz ist weniger als oder gleich 0.60, bestimmt durch Abtastung bei einer Frequenz von 0.01 bis 100Hz mittels eines Rheometers mit einer flachen Geometrie von 40mm, einer Luftspalte von 1000 Mikrometer und einer Analysetemperatur von 25°C.

2. Die injizierbare sterile wässrige Formulierung gemäss Anspruch 1, **dadurch gekennzeichnet, dass** das Molekulargewicht der Hyaluronsäure, oder eines ihrer Salze, zwischen 2.5x10⁵Da und 4x10⁶Da liegt.

3. Die injizierbare sterile wässrige Formulierung gemäss Anspruch 1, **dadurch gekennzeichnet, dass** die Konzentration an vernetzter Hyaluronsäure, oder eines ihrer Salze, zwischen 1% und 3% (Masse/Volumen) liegt.

4. Die injizierbare sterile wässrige Formulierung gemäss Ansprüchen 1 bis 3, **dadurch gekennzeichnet, dass** die Konzentration an Hydroxyapatit zwischen 20% und 60% (Masse/ Volumen), vorzugsweise zwischen 30% und 50% liegt.

5. Die injizierbare sterile wässrige Formulierung gemäss irgendeinem der vorhergehenden Ansprüche, **dadurch gekennzeichnet, dass** die Formulierung zudem ein oder mehrere keramische Materialien wie Tricalciumphosphat umfasst.

6. Die injizierbare sterile wässrige Formulierung gemäss Anspruch 5, **dadurch gekennzeichnet, dass** das keramische Material Tricalciumphosphat ist.

7. Die injizierbare sterile wässrige Formulierung gemäss irgendeinem der vorhergehenden Ansprüche, **dadurch gekennzeichnet, dass** die Formulierung zudem ein Anästhetikum oder mehrere Anästhetika umfasst.

8. Die injizierbare sterile wässrige Formulierung gemäss Anspruch 7, **dadurch gekennzeichnet, dass** ein Anästhetikum oder mehrere Anästhetika ausgewählt wird/werden aus der Gruppe bestehend aus: Lidocain alleine oder in Kombination mit Adrenalin, Procain, Etidocain alleine oder in Kombination mit Adrenalin, Articain alleine oder in Kombination mit Adrenalin, Mepivacain, Pramocain, quinisocain oder einem oder mehreren dieser Anästhetika.

9. Die injizierbare sterile wässrige Formulierung gemäss Anspruch 8, **dadurch gekennzeichnet, dass** das Anästhetikum Lidocainhydrochlorid ist.

10. Die injizierbare sterile wässrige Formulierung gemäss irgendeinem der vorhergehenden Ansprüche, **dadurch gekennzeichnet, dass** die Formulierung zudem ein Antioxidans oder mehrere Antioxidantien umfasst.

11. Die injizierbare sterile wässrige Formulierung gemäss Anspruch 10, **dadurch gekennzeichnet, dass** ein Antioxidans oder mehrere Antioxidantien aus der Polyolfamilie ausgewählt wird/werden.

12. Die injizierbare sterile wässrige Formulierung gemäss Anspruch 10, **dadurch gekennzeichnet, dass** die Polyols Sorbitol, Glycerol, Mannitol oder Propylenglycol umfassen.

13. Die injizierbare sterile wässrige Formulierung gemäss irgendeinem der vorhergehenden Ansprüche, **dadurch gekennzeichnet, dass** die Formulierung zudem einen oder mehrere Wachstumsfaktoren umfasst, wie diejenige aus der Familie der knochenmorphogenetischen Proteine (BMP) und/oder transformierenden Wachstumsfaktoren-β (TGF-β).

14. Die injizierbare sterile wässrige Formulierung gemäss irgendeinem der vorhergehenden Ansprüche, zur Verwendung in orthopädischer, dentaler, Kiefer- und Gesichts-, HNO-, gastroenterologischer, urologischer oder gynäkologischer Chirurgie.

15. Die injizierbare sterile wässrige Formulierung gemäss irgendeinem der vorhergehenden Ansprüche, zur Verwendung in der Füllung und/oder Wiederherstellung von Volumen und/oder zum Ersetzen von biologischen Geweben.

16. Set mit der injizierbaren sterilen wässrigen Formulierung gemäss irgendeinem der Ansprüche 1 bis 15.

17. Set gemäss Anspruch 16, in der Form einer Spritze, einer Ampulle oder eines Kolbens.

18. Verfahren zur Herstellung einer injizierbaren sterilen wässrigen Formulierung, mit den folgenden Schritten: a) Aufbereitung einer ersten Mischung mit mindestens 1 bis 4 Gewichtsprozenten vernetzter Hyaluronsäure oder eines ihrer Salze, durch Bildung von kovalenten Bindungen zwischen den Ketten des besagten Biopolymers unter Verwendung von bi- oder polyfunctionellen Molekülen, worin die durchgeführte Vernetzung es ermöglicht, ein Gel auf Basis von vernetzter Hyaluronsäure mit einer so-genannten monophasischen oder kohäsiven Struktur zu erhalten, b) Reinigung der besagten ersten Mischung, c) dann Zuführung des Hydroxyapartits in einer Konzentration zwischen 10% und 70% (Masse/Volumen), indem es auf homogene Weise in das Gel auf Basis von vernetzter Hyaluronsäure verteilt wird, worin das besagte Hydroxyapartit in der Form von Partikeln vorliegt, deren Durchschnittsgrösse weniger als oder gleich 80µm oder weniger als oder gleich 500nm beträgt, d) Aufbereitung des so erhaltenen Gels in einer gebrauchsfertigen Form, e) Sterilisierung des Produkts unter feuchter Hitze.

## Claims

1. Injectable sterile aqueous formulation, sterilized under damp heat, used for therapeutic purposes, in the form of a particulate cohesive viscoelastic gel comprising:
i) cross-linked hyaluronic acid, or one of its salts, at a concentration comprised between 1% and 4% (mass/volume), wherein the performed reticulation makes it possible to obtain a gel based on cross-linked hyaluronic acid of a so-called cohesive structure, and
ii) hydroxyapatite, at a concentration comprised between 10% and 70% (mass/ volume), wherein said hydroxyapatite is in the form of particles whose average size is less than or equal to 80µm or less than or equal to 500nm;
wherein said injectable sterile aqueous formulation having viscoelastic properties such as Tanδ at the frequency of 1Hz is lower than or equal to 0.60 as determined by performing a scanning at the frequency of 0.01 to 100Hz using a rheometre with a flat geometry of 40mm, an air gap of 1000 micrometres and an analysis temperature of 25°C.

2. The injectable sterile aqueous formulation according to claim 1, **characterized in that** the molecular mass of the hyaluronic acid, or one of its salts, is comprised between 2.5x10⁵Da and 4x10⁶Da.

3. The injectable sterile aqueous formulation according to claim 1, **characterized in that** the concentration of cross-linked hyaluronic acid, or one of its salts, is comprised between 1% and 3% (mass/volume).

4. The injectable sterile aqueous formulation according to claims 1 to 3, **characterized in that** the concentration of hydroxyapatite is comprised between 20% and 60% (mass/ volume), preferably between 30% and 50%.

5. The injectable sterile aqueous formulation according to any of the preceding claims, **characterized in that** the formulation further comprises one or several ceramic materials such as tricalcium phosphate.

6. The injectable sterile aqueous formulation according to claim 5, **characterized in that** the ceramic material is tricalcium phosphate.

7. The injectable sterile aqueous formulation according to any of the preceding claims, **characterized in that** the formulation further comprises one or several anaesthetics.

8. The injectable sterile aqueous formulation according to claim 7, **characterized in that** one or several anaesthetics is chosen from the group comprising lidocaine alone or in combination with adrenalin, procaine, etidocaine alone or in combination with adrenaline, articaine alone or in combination with adrenaline, mepivacaine, pramocaine, quinisocaine or one or several of these anaesthetics.

9. The injectable sterile aqueous formulation according to claim 8, **characterized in that** the anaesthetic is lidocaine hydrochloride.

10. The injectable sterile aqueous formulation according to any of the preceding claims, **characterized in that** the formulation further comprises one or several antioxidants.

11. The injectable sterile aqueous formulation according to claim 10, **characterized in that** one or several antioxidants is chosen from the polyol family.

12. The injectable sterile aqueous formulation according to claim 10, **characterized in that** the polyols comprise sorbitol, glycerol, mannitol or propylene glycol.

13. The injectable sterile aqueous formulation according to any of the preceding claims, **characterized in that** the formulation further comprises one or several growth factors, such as those from the family of bone morphogenetic proteins (BMP) and/or transforming growth factors-β (TGF-β).

14. The injectable sterile aqueous formulation according to any of the preceding claims, used in orthopaedic, dental, maxillofacial, ENT, gastroenterological, urologic or gynaecologic surgery.

15. The injectable sterile aqueous formulation according to any of the preceding claims, used for filling in and/or repairing volumes and/or replacing biological tissues.

16. Kit comprising the injectable sterile aqueous formulation according to any of the claims 1 to 15.

17. Kit according to claim 16, presenting in the form of a syringe, ampoule or vial.

18. Method for manufacturing an injectable sterile aqueous formulation, comprising the steps of: a) preparing a first mixture comprising at least 1% to 4% by weight of cross-linked hyaluronic acid or one of its salts, by formation of covalent bonds between the chains of said biopolymer using bi- or poly-functional molecules, wherein the performed reticulation makes it possible to obtain a gel based on cross-linked hyaluronic acid of a so-called monophasic or cohesive structure, b) purifying said first mixture, c) then adding the hydroxyapatite at a concentration comprised between 10% and 70% (mass/volume) by dispersing it in a homogeneous manner in the gel based on cross-linked hyaluronic acid, wherein said hydroxyapatite is in the form of particles whose average size is less than or equal to 80µm or less than or equal to 500nm, d) putting the gel thus obtained in a form ready for use, e) sterilizing the product under damp heat.
